# EUROPEAN PATENT SPECIFICATION

(11) **EP 3 846 864 B1**
(45) Date of publication and mention of the grant of the patent: **01.11.2023**
(21) Application number: 18793263.7
(22) Date of filing: 06.09.2018
(51) Int. Cl.: A61L 27/18, A61L 27/56

(54) **MEDICAL DEVICE FOR USE IN THE REGENERATION OF AN INJURED NERVE**
MEDIZINISCHE VORRICHTUNG ZUR VERWENDUNG BEI DER REGENERATION EINES VERLETZTEN NERVS
DISPOSITIF MÉDICAL DESTINÉ À ÊTRE UTILISÉ DANS LA RÉGÉNÉRATION D'UN NERF LÉSÉ

(43) Date of publication of application: 14.07.2021
(73) Proprietor: Bio-On S.p.A., 40016 San Giorgio Di Piano (BO) (IT)
(72) Inventor: GEUNA, Stefano, 10043 Orbassano (TO) (IT); MONACO, Ilaria, 40016 San Giorgio Di Piano (BO) (IT); MATURI, Mirko, 40136 Bologna (IT); SAETTONE, Paolo, 40016 San Giorgio Di Piano (BO) (IT); CIFELLI, Mario, 40016 San Giorgio Di Piano (BO) (IT); COMES FRANCHINI, Mauro, 40016 San Giorgio Di Piano (BO) (IT)
(74) Representative: Bottero, Carlo
(86) International application number: PCT/IB2018/056803
(87) International publication number: WO 2020/049337

(56) References cited:
- WO-A1-2005/002472
- WO-A1-2013/154780
- WO-A1-2017/153619
- US-A1- 2014 277 572

## Description

The present invention relates to a medical device for use in the regeneration of an injured nerve. In particular, the present invention relates to a medical device for use in the regeneration of an injured nerve, in which said device comprises a tubular membrane.

It is well known that the traumatic lesions of the peripheral nerves are an event of considerable clinical importance due to the high incidence: it has been estimated a number of cases equal to about 300,000 a year, only in Europe, and more than a million cases a year all over the world.

Most of the traumatic injuries of the peripheral nerves occur at the level of the upper limb and the causes can be many: car and motorcycle accidents, domestic falls, accidents at work or following sporting activities. Iatrogenic nerve injuries can also occur during surgeries, anesthesia injections, chemotherapy or radiotherapy for the treatment of breast, head and neck cancers. Finally, the obstetric lesions of the brachial plexus have an incidence of 1.24 cases every 1000 births.

The consequences of a nerve injury can be very serious due to the partial or total loss of the motor, sensitive and autonomic function, interfering with many aspects of everyday life. The occurrence of secondary problems, such as neuropathic pain, dysaesthesia and cold intolerance, further aggravates the condition. Finally, nerve injuries also have a significant economic impact on society in terms of health care costs and long periods of absence from work due to illness.

Although the ability of the peripheral nerve to regenerate independently has been recognized for more than a century, clinical and experimental evidences show that such regeneration is often unsatisfactory, especially following serious injury.

In the case of simple lesions, in which there is no loss of substance, the technique used in surgery is direct suture in the absence of tension. If there is a loss of nervous substance and direct suture is not possible, it is necessary to resort to alternative strategies. Polyhydroxyalkanoate (PHA) based tubular membranes for nerve regeneration are known from WO 2017/153619 A1 and WO 2013/154780 A1 discloses chitosan nerve guides comprising Ag nanowires.

Nowadays, the reference standard (the so-called gold standard) in the treatment of these lesions is the autologous nerve graft. However, this technique has limitations such as the need for double surgical access with inevitable damage at the donor site, and the unavailability of large quantities of nerve grafts. There is therefore a strong need to find innovative therapies.

The Applicant has therefore posed the problem of developing a device for use in the regeneration of an injured nerve, without resorting to autologous graft, using a tubular membrane that has the ability to promote the regeneration of nerve fibers in a relatively short time, so as to favor functional recovery without inducing inflammatory or rejection reactions.

The Applicant has now found that this problem, and others which will be better illustrated hereafter, can be solved by using, for the regeneration of an injured nerve, a medical device comprising a tubular membrane in turn comprising polyhydroxyalkanoate (PHA) fibers, produced by electrospinning, containing 3-hydroxybutyrate monomer units, as defined in claims 1 and 12.

In a first aspect thereof, the present invention therefore relates to a medical device comprising a tubular membrane having openings adapted to receive the ends of an injured nerve and a lumen to allow the regeneration of said nerve, wherein said tubular membrane comprises fibers of polyhydroxyalkanoate (PHA) containing 3-hydroxybutyrate monomer units, said fibers having an average diameter comprised from 500 nm to 2000 nm, wherein the tubular membrane further comprises Ag nanowires coated with a binding agent selected from: a thiol R-SH, wherein R is a saturated or unsaturated, aliphatic and/or aromatic hydrocarbon group, having from 8 to 38 carbon atoms, optionally substituted with functional groups selected from: ester, amide, carboxyl, hydroxyl, amine groups; and a protein biopolymer containing -SH groups.

Preferably, the medical device according to the invention comprises a tubular membrane having two openings which lie on planes substantially perpendicular to the longitudinal axis of the membrane itself. Between these openings there is a passing lumen within which the regeneration of the injured nerve occurs.

Analyzes carried out by the Applicant (described in detail hereafter) have in fact surprisingly shown that the medical device according to the invention has the advantages of not causing inflammation of the surrounding tissues surrounding the injured nerve, thus demonstrating a high biocompatibility, and at the same time exhibiting high ability to induce the regeneration of the injured nerve, providing a valid support for the maturation of the newly formed nerve fibers.

The area adjacent to the grafting point of the medical device has neither symptoms due to inflammatory reactions nor the formation of fibrous scar tissue. Furthermore, after one month from the grafting of the device, a large number of axons in the regenerative phase and various blood vessels can be seen inside the lumen of the tubular membrane. At three months from the graft, medium-sized nerve fibers having a high degree of myelination are visible.

In a preferred embodiment, the PHA fibers containing 3-hydroxybutyrate monomer units are advantageously oriented in a direction substantially parallel to the longitudinal axis of the tubular membrane.

The Applicant considers that the nerve regeneration induced by the medical device according to the present invention is attributable, at least in part, to the fact that the PHA containing 3-hydroxybutyrate monomer units is a piezoelectric polymer: this feature, in particular associated with the substantially parallel orientation of the fibers, stimulates and orients the regeneration of the nerve correctly.

As indicated above, the PHA fibers have an average diameter comprised from 500 nm to 2000 nm, preferably comprised from 800 nm to 1200 nm.

Preferably, the PHA fibers are produced by electrospinning. In the context of the present description and of the accompanying claims, "electrospinning" means a spinning method, known in the art, which exploits the interaction that is created between a polymeric solution and an external electric field. Electrospinning makes it possible to produce very small diameter fibers, generally of the order of tens or hundreds of microns, with high speed and accurate process control, a result difficult to obtain with common fiber production techniques by extrusion spinning.

An electrospinning system mainly comprises a pump connected to a syringe containing the polymeric solution, a spinneret connected to the syringe, a high voltage source and a manifold. Due to the action of the pump, the polymeric solution is pushed outside the syringe, towards the manifold, passing through the spinneret with a constant and controllable flow. When a high potential difference (usually between 1 and 30 kV) is applied between the spinneret and the manifold, a drop of polymer is generated at the tip of the spinneret. As the difference in potential increases, the drop is subjected to increasing repulsive forces between its surface charges and the electrostatic forces exerted by the external electric field, up to the distortion of the drop itself with the formation of a cone commonly known as Taylor's cone. As soon as the electric field exceeds a critical value, specific for each polymeric solution, the electrostatic forces prevail over the surface tension leading to the formation of a polymer fiber or wire.

Advantageously, the tubular membrane of the medical device according to the invention also comprises silver nanowires (Ag nanowires, AgNWs).

Preferably, the tubular membrane comprises from 0.2% to 5% by weight, more preferably from 0.5% to 2.5% by weight, with respect to the overall weight of the membrane, of Ag nanowires.

Preferably, the Ag nanowires have an average diameter comprised from 10 nm to 300 nm, more preferably comprised from 30 nm to 100 nm.

Preferably, the Ag nanowires have an average length comprised from 10 um to 200 um, preferably comprised from 30 um to 100 um.

Preferably, the Ag nanowires are oriented in a direction substantially parallel to the longitudinal axis of the tubular membrane.

The Applicant believes that the presence of Ag nanowires inside the tubular membrane increases the electrical conductivity of the device itself, and this further enhances the regenerative capacity of the nerve fibers.

Analyzes carried out by the Applicant (described in detail hereafter) have also surprisingly shown that the medical device according to the present invention, in the embodiment comprising the Ag nanowires, favors a type of regeneration which initially develops near the inner wall of the tubular membrane and not at the center of the lumen as occurs in the absence of such Ag nanowires. In other words, the presence of Ag nanowires in the tubular membrane attracts the regenerating nervous tissue, thus favoring the regeneration pathway thereof.

The Ag nanowires are coated by a binding agent. This agent has the main function of increasing the compatibility between Ag nanowires and PHA fibers, so as to favor the integration of Ag nanowires with PHA. The binding agent generally has a hydrocarbon chain, of sufficient length to favor the interaction with the PHA, functionalized with at least one group that is capable of binding with silver.

Preferably, the binding agent is a thiol R-SH, wherein R is a saturated or unsaturated, aliphatic and/or aromatic hydrocarbon group, having from 8 to 38 carbon atoms, optionally substituted with functional groups, such as ester, amide, carboxyl, hydroxyl, amine groups.

In particular, the binding agent is preferably selected from products of general formula (I):

HS-Ph-CO-NH-R₁-CO-OR₂ (I)

wherein:
Ph is a phenyl;
R₁ is a linear or branched C₄-C₂₀ alkylene;
R₂ is a linear or branched C₁-C₄ alkyl.

More preferably, R₁ is a linear C₈-C₁₆ alkylene.

More preferably, R₂ is selected from: methyl, ethyl, n-propyl, i-propyl, b-butyl, i-butyl, t-butyl.

Even more preferably, the binder is a binder of formula (II):

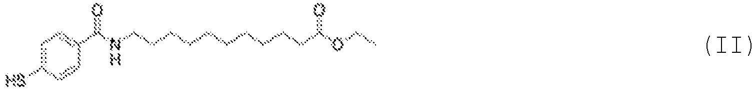

Ag nanowires which can be used in the device according to the present invention are known products and commercially available from various manufacturers, including American Elements, Novarials Corp. and others.

Ag nanowires are generally produced by precipitation of silver from a polyvinyl pyrrolidone (PVP) solution in a suitable solvent, for example a polyol such as ethylene glycol or glycerol, which acts as a reducing agent, to which an aqueous solution of a silver salt, in particular silver nitrate, is added. In order to control the concentration of metallic Ag produced and in particular to promote the formation of Ag nanowires and prevent the formation of aggregates having different shapes from that to nanowires, a halide, such as chloride or bromide, is preferably added to the reaction solution.

The solution thus obtained is heated to a temperature between 120°C and 160°C and maintained at this temperature without stirring for a time ranging from 2 to 8 hours. After cooling at room temperature, a suitable solvent is added, e.g. acetone, which induces the precipitation of the silver nanowires. The latter are then separated from the solvent, for example by centrifugation.

Following this production process, the obtained Ag nanowires are coated with PVP, which is preferably substituted with a binding agent as described above, so as to improve compatibility with the PHA. The binding agent is capable of replacing the PVP on the surface of the nanowires, thus forming an ordered top monolayer (self-assembled monolayer) by π-stacking interactions and Van Der Waals forces.

Preferably, Ag nanowires coated with a binding agent comprise from 60% to 90% by weight, more preferably from 65% to 80% by weight, of Ag and from 10% to 40% by weight, more preferably from 20% to 35% by weight of the binding agent.

For water-based applications, Ag nanowires can be advantageously coated with a protein biopolymer containing -SH groups, usually present thanks to the amino acid cysteine which is part of the polypeptide chain of numerous natural proteins, in particular animal gelatin. Such -SH groups are capable of improving the biocompatibility of Ag nanowires with PHA and/or with the tissues with which they come into contact after implantation of the device according to the present invention. Animal gelatin generally consists of a mixture of water-soluble proteins and peptides and is a product obtained by extraction in hot aqueous solution from animal connective tissues, by hydrolysis. Due to the presence of a large number of polar functional groups in the amino acids that make up the protein chains of gelatin (especially amino and carboxylic groups) and due to the presence of a small percentage of cysteine, gelatin is capable of generating interactions with Ag nanowires, which are more stable than those of PVP, whereby gelatin is capable of replacing the latter on the surface of Ag nanowires.

Preferably, Ag nanowires coated with a protein biopolymer containing -SH groups comprise from 70% to 98% by weight, more preferably from 80% to 95% by weight, of Ag and from 2% to 30% by weight, more preferably from 5% to 20% by weight of the protein biopolymer containing -SH groups.

According to the purposes of the present invention, the PHA containing 3-hydroxybutyrate monomer units is preferably a poly-3-hydroxybutyrate homopolymer (P3HB) or a copolymer containing at least 20% moles of 3-hydroxybutyrate monomer units, the remainder being hydroxyalkanoate monomer units other than 3-hydroxybutyrate.

In general, PHAs suitable for the present invention are homopolymers consisting of 3-hydroxybutyrate monomer units:

-O-CH(CH₃)-CH₂-CO- (III)

or copolymers of 3-hydroxybutyrate monomer units with at least one hydroxyalkanoate monomer unit having the formula

-O-CHR₁-(CH₂)ₙ-CO- (IV)

wherein:
R₁ is selected from: -H and C₁-C₁₂ alkyl;
n is zero or an integer in the range of 1 to 6, and is preferably 1 or 2; with the proviso that, when R₁ is methyl, n is different from 1. Preferably, R₁ is methyl or ethyl. Preferably, n is 1 or 2.

In the case of a copolymer, it preferably contains at least 20% moles, more preferably at least 30% moles, of 3-hydroxybutyrate monomer units, the remainder being hydroxyalkanoate monomer units other than 3-hydroxybutyrate. In such copolymers, the quantity of 3-hydroxybutyrate monomer units is generally equal to or less than 99% moles, preferably equal to or less than 90% moles.

Particularly preferred hydroxyalkanoate monomer units other than 3-hydroxybutyrate are those deriving from: 4-hydroxybutyrate, 3-hydroxyvalerate, 3-hydroxyhexanoate, 3-hydroxyoctanoate, 3-hydroxyundec-10-enoate, 4-hydroxyvalerate.

In the case of copolymers of PHA, they are preferably selected from: poly(3-hydroxybutyrate-co-3-hydroxyvalerate) (P3HBV), poly(3-hydroxybutyrate-co-3-hydroxyhexanoate) (P3HBH), poly(3-hydroxybutyrate-co-4-hydroxybutyrate), poly(3-hydroxybutyrate-co-3-hydroxyvalerate-co-4-hydroxyvalerate (P3HVV), or mixtures thereof.

According to the purposes of the present invention, preferably the PHA is a copolymer containing at least 20% moles of 3-hydroxybutyrate monomer units, the remainder being monomer units of 3-hydroxyvalerate. Even more preferably, the PHA is poly(3-hydroxybutyrate-co-3-hydroxyvalerate) (P3HBV).

PHAs suitable for the present invention preferably have a weight average molecular weight (Mw) comprised in the range from 5,000 to 1,500000 Da, more preferably from 100,000 to 1,000,000 Da. The weight average molecular weight can be determined according to known techniques, in particular by means of GPC ("Gel Permeation Chromatography") analysis.

The Applicant believes that the use of PHA fibers containing 3-hydroxybutyrate monomer units, and in particular poly-3-hydroxybutyrate fibers (P3HB), ensures a substantially complete resorption of the fibers by the organism in which the device has been implanted, without any toxic or harmful effect.

In fact, when the PHA containing 3-hydroxybutyrate monomer units is metabolized within the organism, the 3-hydroxybutyric acid monomer is released, which, due to the presence of an OH group on the carbon in beta position, undergoes a dehydration reaction catalyzed by some enzymes, resulting in the formation of crotonic acid (an alpha-beta-unsaturated organic acid). Within the organism, the crotonic acid undergoes a reaction of addition of hydrogen to the double bond with consequent formation of butyric acid (a "Short Chain Fatty Acid" SCFA that is easily assimilated by the body). This metabolic pathway is impossible with other monomers derived from other PHAs, such as 4-hydroxybutyric acid which is generated as a result of the metabolization of poly-4-hydroxybutyrate (P4HB): in fact, in 4-hydroxybutyric acid the OH group is positioned on the gamma carbon and not on the beta carbon, this makes it impossible to form crotonic acid by dehydration.

The medical device according to the invention can be made, in particular, by electrospinning, with dimensions suitable for surgical needs, that is to say, depending on the length and the extent of the nerve injury to be treated. Generally, the medical device may have a length comprised from 3 to 50 mm, an inner diameter comprised from 0.5 to 5.0 mm, an outer diameter comprised from 0.7 to 5.3 mm. The tubular membrane may have a thickness comprised from 50 to 300 um, preferably comprised from 100 to 150 um.

According to another aspect thereof, the present invention relates to a method for producing a medical device as described above, which comprises:
- preparing a spinning solution by solubilization of polyhydroxyalkanoate (PHA) containing 3-hydroxybutyrate monomer units in an organic solvent;
- subjecting the spinning solution to an electrospinning process on a rotating support so as to obtain a tubular membrane.

In the case in which a tubular membrane must be produced in which the PHA fibers are combined with Ag nanowires, the latter are dispersed in the spinning solution, so as to achieve a co-deposition of PHA fibers and Ag nanowires on the rotating support which ensures a substantially homogeneous distribution of the nanowires inside the tubular membrane. The rotating support generally has a substantially cylindrical shape and is connected to a motor which allows an accurate control of the rotation speed.

The organic solvent which can be used for the preparation of the spinning solution may be selected from those in which the PHA is soluble and among those that also have features of good electrical conductivity and polarity, for example 1,1,1,3,3,3-hexafluoro-2-propanol (HFIP), chloroform, N,N-dimethylformamide (DMF), or mixtures thereof.

In the spinning solution, the PHA containing 3-hydroxybutyrate monomer units preferably has a concentration comprised from 1% to 20% w/v, more preferably comprised from 2 to 10% w/v. When present, the Ag nanowires are present in the spinning solution in a concentration preferably comprised from 0.05% to 2.0% w/v, more preferably comprised from 0.1% to 1.0% w/v.

According to a further aspect thereof, the present invention also relates to a method for the regeneration of an injured nerve comprising: inserting each of the two ends of the injured nerve into a medical device comprising a tubular membrane having openings adapted to receive the ends of the injured nerve and a lumen to allow the regeneration of said nerve, wherein said tubular membrane comprises fibers of polyhydroxyalkanoate (PHA) containing 3-hydroxybutyrate monomer units, said fibers having an average diameter comprised from 500 nm to 2000 nm. Preferably, the two ends of the injured nerve are inserted into the tubular membrane itself.

The following embodiment examples are provided purely for illustrative purposes of the present invention and should not be interpreted as limiting the scope of protection defined by the accompanying claims.

### EXAMPLE 1: Production of the P3HB tubular membrane.

The purified P3HB was dissolved in HFIP (1,1,1,3,3,3-hexafluoro-2-propanol) so as to obtain a spinning solution with a concentration of 4% w/v. The solution was stirred on a plate at 800 rpm at a temperature of 80°C for about 2 hours.

Thereafter, the solution was cooled, poured into a 10 mL plastic syringe and placed inside the electrospinning system to be processed. The deposition of the fibers was carried out on a steel cylindrical support (spindle) placed in rotation by an electric motor.

The following parameters were used for the electrospinning process:
Spinning solution: purified P3HB dissolved in HFIP at 4% w/v;
solution volume processed: 1 mL;
solution rate: 1 mL/hour;
translational speed of the spinneret along the spindle: 3 mm/s;
distance between the tip of the spinneret and the spindle (gap): 15 cm;
voltage applied to the electrodes: 20 kV;
spindle rotation speed: 200 rpm;
diameter of the spinneret outlet hole: 2.15 mm;
spindle diameter: 1.5 cm;
auxiliary electrode (16 cm x 16 cm) placed about 1 cm from the circular ground electrode.

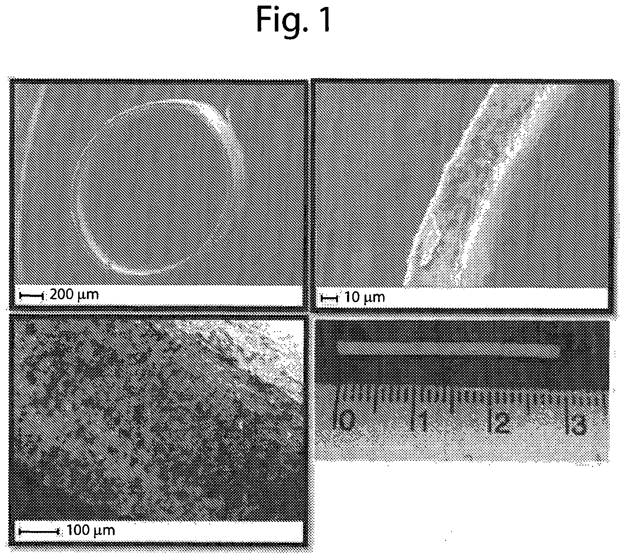

Figure 1 shows the images of the tubular membrane, obtained by electronic scanning microscopy (SEM).

### EXAMPLE 2: Ag nanowires production.

In a 100 mL flask, 0.485 g of polyvinyl pyrrolidone (PVP) were dissolved in ethylene glycol (40 mL) while the solution was stirred to complete dissolution. Thereafter, 0.6 mL of an anhydrous ferric chloride solution in glycerol (11.2 mM) and 0.725 g of silver nitrate were added to the solution, stirring to complete dissolution.

The solution was placed in a pre-heated oven at 140°C and kept at this temperature for 4 and a half hours without stirring and protected from light. Then, the solution was allowed to cool to room temperature, then acetone (50 mL) was added to induce the precipitation of Ag nanowires.

Ag nanowires were separated from the suspension by centrifugation, with cycles of 15 minutes each at 4500 rpm. The nanowires thus obtained were purified from possible impurities by washing them in a centrifuge with ethanol (2 x 50 mL) and, finally, with purified and deionized water (2 x 50 mL times). The resulting precipitate was dispersed in water and filtered with woven or nylon filters characterized by pores with a diameter of 30-50 um, in order to eliminate the too small nanowires, having an unsuitable length. The final suspension was diluted up to 100 mL with water.

The concentration of Ag in the suspension of nanowires thus obtained was determined by atomic absorption spectroscopy (AAS), obtaining a value equal to 6.8 g/L of Ag which corresponds to a yield of 94%. The concentration of PVP remaining in the final suspension, after purification, was determined by thermogravimetric analysis (TGA) and was found to be equal to 52% of the total mass.

Ag nanowires thus obtained had an average length of 60 um and an average diameter of 100 nm.

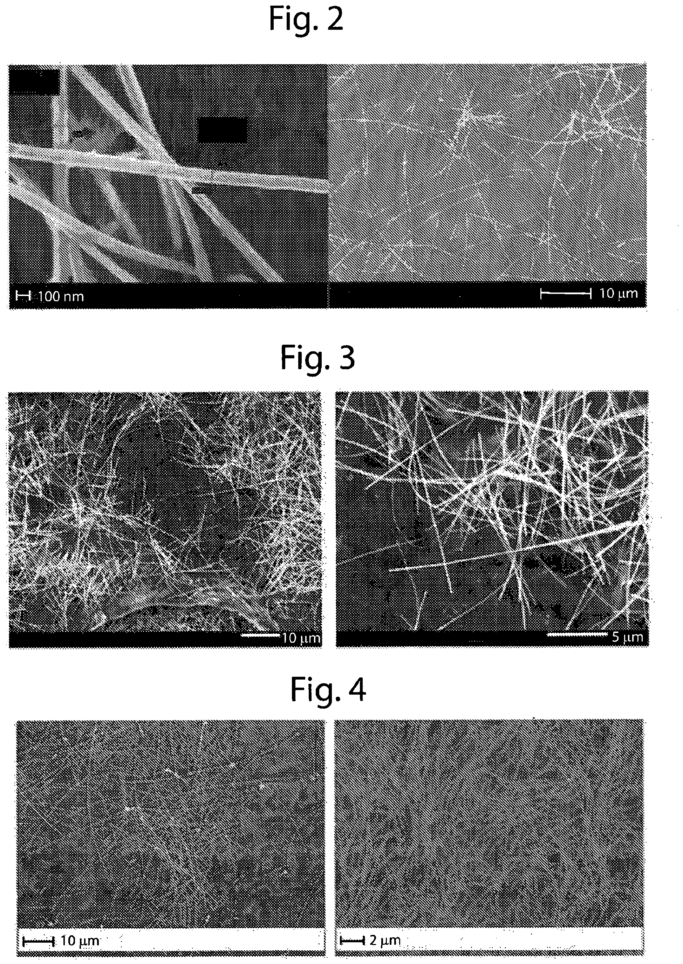

Figure 2 shows the images of Ag nanowires, obtained by electronic scanning microscopy (SEM).

### EXAMPLE 3: Coating of Ag nanowires with a binding agent.

To the suspension of Ag nanowires (PVP-coated) in water (10 g/L), obtained according to Example 2, an equal volume of a solution of the binding agent of formula (II) (11-(4-mercaptobenzamide)ethyl undecanoate) in ethanol (10 g/L) was added dropwise. The resulting mixture was left under slow stirring by means of a Vortex mixer for 16 hours at room temperature. At the end, the nanowires were isolated by centrifugation (3000 rpm for 10 minutes) or alternatively they are filtered with woven or nylon filters characterized by pores with a diameter of 30-50 um.

Thereafter, the Ag nanowires thus treated were purified by ethanol washes to remove the excess binder, and finally they were suspended in chloroform.

The concentration of Ag in the suspension thus obtained was determined by atomic absorption spectroscopy (AAS), obtaining a value equal to 4.9 g/L. The sample was also analyzed by thermogravimetric analysis (TGA) obtaining a composition equal to 70% by weight of Ag and 30% by weight of binder of formula (II) .

The Ag nanowires were finally dried out and stored at room temperature protected from light and moisture.

Figure 3 shows the images of Ag nanowires coated with the binding agent, obtained by electronic scanning microscopy (SEM).

### EXAMPLE 4: Coating of Ag nanowires with animal gelatin.

A solution of pork gelatin (240 Bloom degrees) was prepared in water at 40°C, to which an equal volume of suspension in water (20 g/L) of Ag nanowires obtained according to Example 2 was added dropwise, so as to have, in the final mixture, a gelatin concentration equal to 35 g/L and an Ag concentration equal to 10 g/L. The mixture was stirred for 16 hours at 40 °C. At the end of stirring, the Ag nanowires were isolated from PVP and from excess gelatin by centrifugation and repeated washes with warm water (40°C). The final product was then stored in a suspension at a concentration of 40 g/L of Ag (concentration obtained following the removal of excess gelatin and removal of the initial PVP). Following thermogravimetric analysis (TGA), it was found that Ag constituted 90% of the dry mass of the suspension, while gelatin constituted the remaining 10%. The final product was then dried by freeze-drying and stored away from light, moisture and at room temperature.

Figure 4 shows the images of Ag nanowires coated with animal gelatin, obtained by electronic scanning microscopy (SEM).

### EXAMPLE 5: Production of the P3HB and Ag nanowires tubular membrane.

The purified P3HB was dissolved in HFIP (1,1,1,3,3,3-hexafluoro-2-propanol) so as to obtain a spinning solution with a concentration of 4% w/v. The solution was stirred on a plate at 800 rpm at a temperature of 80°C for about 2 hours.

Once the polymer was dissolved, 0.1 mL of a suspension of Ag nanowires in HFIP (92.7 mM, 10 g/L) were added, prepared and treated with the binding agent of formula (II) as described in Example 3. The solution thus added was stirred on a plate at 800 rpm for about 2 hours at room temperature.

The solution was then poured into a 10 mL plastic syringe and placed inside the same electrospinning system used in Example 1.

The following parameters were used for the electrospinning process:
spinning solution: purified P3HB dissolved in HFIP at 4% w/v + 0.2% w/v Ag in HFIP;
solution volume processed: 5 mL;
solution rate: 3 mL/hour;
translational speed of the spinneret along the spindle: 3 mm/s;
distance between the tip of the spinneret and the spindle (gap): 15 cm;
voltage applied to the electrodes: 15 kV;
spindle rotation speed: 300 rpm;
diameter of the spinneret outlet hole: 2.15 mm;
spindle diameter: 1.5 cm;
auxiliary electrode (16 cm x 16 cm) placed about 1 cm from the circular ground electrode.

The following composition of the tubular membrane thus obtained was determined by atomic absorption spectroscopy (AAS): 98.5% by weight of P3HB and 1.5% by weight of Ag.

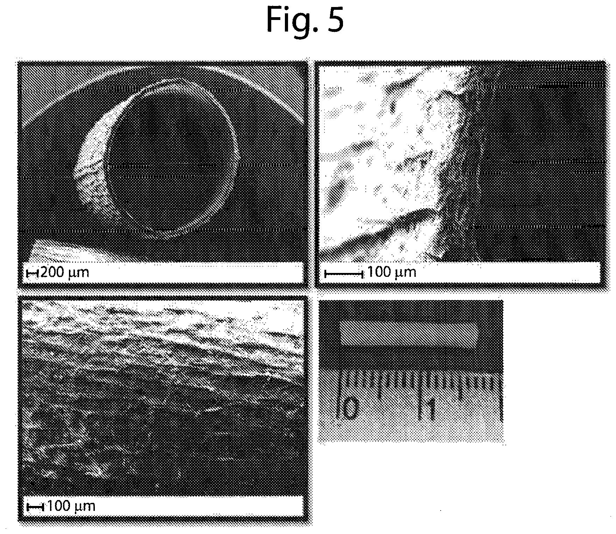

Figure 5 shows the images of the tubular membrane, obtained by electronic scanning microscopy (SEM).

### EXAMPLE 6: Pre-clinical study of medical devices.

### (a) Animals and surgery.

Nerve regeneration analysis conducted *in vivo* was performed under general anesthesia using Zolazepam (Zoletil, Virban) + Xylazine (Bayer) by intraperitoneal injection (40 mg/kg + 5 mg/kg), on adult female Wistar rats, weighing about 200 g. The median nerves were repaired by medical devices comprising a tubular membrane comprising P3HB (with or without silver nanowires) or by autotransplantation and collected 2 or 4 weeks after surgery, as follows:
a) the nerves were repaired with a tubular membrane comprising P3HB (n = 6), or with a tubular membrane comprising P3HB and silver nanowires (n = 4) or by autotransplantation (n = 3) and the regenerated nerves were collected 2 weeks after surgery;
b) the nerves were repaired with a tubular membrane comprising P3HB (n = 6), or with a tubular membrane comprising P3HB and silver nanowires (n = 4) or by autotransplantation (n = 3) and the regenerated nerves were collected 4 weeks after the surgery.

The median nerve was injured and a 10 mm long tubular membrane comprising P3HB (with or without silver nanowires) was used to bridge the nerve injury by inserting 1 mm of each of the two nerve ends within the membrane. The nerve guide was sutured with a 9/0 epineurial point at each end.

In the experimental autotransplant group, a 10 mm nerve segment was cut, inverted (distal - proximal) and sutured at the ends of the injured nerve with three 9/0 epineural points.

On days 0, 1, 2 and 3 from surgery, analgesic therapy with Rymadil (4 mg/kg, Zoetis Italy) was administered subcutaneously, while on days 2 and 5 after surgery, antibiotic treatment was administered by intramuscular injection (Rubrocylline 0.05 ml/500 g, MSD animal health).

2 and 4 weeks after the surgery, the rats were sacrificed by anesthesia overdose with Zoletil + Xylazine (> 60 mg/kg and > 10 mg/kg) by intraperitoneal injection and the regenerated nerves were collected and analyzed.

### (b) Fixation protocol for immunohistochemistry and confocal microscopy.

Regenerated nerve samples, together with the medical device comprising a tubular membrane comprising P3HB, were fixed by immediate immersion in 4% paraformaldehyde in PBS (saline phosphate buffer) (Sigma) for 2-3 hours at 4°C. The nerve samples were stored until the inclusion procedure in PBS at 4 °C.

### (c) Cryo-embedding protocol.

The samples were rehydrated with PBS (Sigma) and cryo-protected by three passages in increasing solutions of sucrose (Sigma) (7.5% for 1 hour, 15% for 1 hour, 30% overnight) in PBS 0.1 M. Thereafter, the samples were kept in a 1:1 solution of 30% sucrose and optimal cutting temperature medium (OCT, ELECTRON MICROSCOPY SCIENCES) for 30 minutes and then embedded in 100% OCT. The samples were stored at -80°C. 10 um thick longitudinal nerve sections were cut and stored at -20°C. For staining, the sections were brought to room temperature and, as soon as they were acclimatized, they were processed.

### (d) Masson's trichrome stain.

A Masson trichrome kit with aniline blue (Bio-Optica) was used for Masson's trichrome stain. Six drops of Weigert's iron hematoxylin (solution A) and six drops of Weigert's iron hematoxylin (solution B) were combined together and used to stain the slides for 10 minutes. Without washing, the slides were then drained and incubated with ten drops of alcoholic picric acid solution for 4 minutes. After washing with distilled water, the sections were stained with ten drops of Ponceau red acid for 4 minutes and washed again in distilled water. Thereafter, ten drops of phosphomolybdic acid solution were added to the section for 10 minutes. Without washing, the slides were dripped and 10 drops of quick dehydrating agent in ethanol were added and cleaned in xylene/Bioclear (Bio-Optica), the slides were mounted in DPX (Fluka).

### (e) Immunohistochemistry and confocal microscopy.

For immunofluorescence, the sections were rinsed in PBS, blocked with normal serum (normal 1% serum in PBS containing 0.1% Triton) (it is recommended to use a normal serum obtained from the same species as the secondary antibody) for 1 hour and then incubated overnight with the primary antibody. For double marking, two different primary antibodies can be used at the same time, provided they are produced in different animal species. After incubation of the primary antibody (or primary antibodies), the sections were washed three times in PBS and incubated for 1 hour in a solution containing the secondary antibody (or secondary antibodies): Alexa 488 a-mouse, Cy3 a-rabbit (Life Technologies) conjugated with fluorophore and selected to recognize the primary antibody/antibodies species. After three washes in PBS, the sections were finally prepared with a Dako fluorescent mounting medium and stored at 4°C before being analyzed.

Both the axon and the glia can be identified by immunohistochemistry using specific antibodies.

The sections were incubated in a solution containing the following primary antibodies, used alone or in pairs:
- anti-NF 200 kDa (monoclonal, mouse, Sigma Aldrich)
- S-100β (polyclonal, rabbit, Sigma Aldrich)

### (f) Resin embedding and optical microscope analysis.

The regenerated nerve samples, together with the medical device comprising a tubular membrane comprising P3HB, were fixed by immediate immersion in 2.5% glutaraldehyde (SIC Società Italiana Chimici) in 0.1 M phosphate buffer (pH 7.4) for 5-6 hours at 4°C. The samples were then post-fixed in 2% osmium tetroxide (SIC Società Italiana Chimici) for 2 hours and dehydrated by passages in ethanol (Sigma Aldrich) from 30% to 100% (5 minutes for each passage). After two 7-minute passages of propylene oxide and overnight in a 1:1 mixture of propylene oxide (Sigma Aldrich) and mixture of Glauerts resins, the samples were embedded in the Glauerts resin mixture (made of equal parts of Araldite M and Araldite Harter, HY 964, Sigma Aldrich). A 0.5% dibutyl phthalate plasticizer (Sigma Aldrich) was added to the resin mixture. For the final phase, the 2% accelerator 964 was added to the resin to facilitate the polymerization of the embedding mixture, at 60°C.

Semi-thin sections (2.5 um thick) were cut using a Ultracut UCT ultramicrotome (Leica Microsystems, Wetzlar, Germany) and stained with 1% toluidine blue for high-resolution optical microscopy examination and design-based stereology. A DM4000B microscope equipped with a DFC320 digital camera and an IM50 image management system (Leica Microsystems, Wetzlar, Germany) was used for section analysis.

### (g) In vivo experiments on nerve regeneration.

The animals inclusion criteria for *in vivo* experiments were based on their weight, age and health condition.

Before collecting the regenerated nerve, each implant site was carefully examined to detect any signs of local tissue alterations.

Thus, to observe the qualitative morphology of the nerve in the early stages of regeneration, both sections were analyzed: longitudinal and transverse. In particular, the longitudinal sections were subjected to Masson's trichrome staining and to immunofluorescence staining to have a macroscopic overview of the initial regeneration phase; the semi-thin cross-sections were stained with toluidine blue and were analyzed by high-resolution optical microscope to study the microscopic behavior of the nerves in regeneration. The images of the cross section were collected inside the graft, both proximally and distally, to follow the regeneration of the nerve along the medical device.

All analyzes were conducted in the short term after the surgery (2 and 4 weeks).

The nerves repaired with the medical device comprising a tubular membrane comprising P3HB and silver nanowires were analyzed by high-resolution optical microscopy analyzing semi-thin transversal sections stained with toluidine blue.

### (h) Qualitative evaluation of scar formation.

On the day of the sacrifice, each implant site was examined for the presence of local tissue alterations with a high magnification surgical microscope. Special attention was paid to identify any signs of inflammation and/or development of scar tissue that could be attributed to the degradation of the implant. All tubular membranes comprising P3HB (with or without silver nanowires) were found at the graft site, covered with a thin layer of connective tissue. A careful peri-implant examination showed no signs of inflammation or scar tissue formation around the membranes.

### (i) Two weeks after the surgery.

The trichrome staining of the longitudinal sections showed the presence of a thin layer of connective tissue surrounding the outer side of the tubular membrane.

After 2 weeks of surgery, several types of cells colonized the lumen of the medical device comprising P3HB (Schwann cells, fibroblasts, immune system cells) and several vessels inside the lumen of the medical device were also identified in cross sections stained with toluidine blue.

The central portion of the medical device has not yet been completely colonized by cells or extracellular matrix, which means that the cells must still migrate to the central portion of the medical device.

In the semi-thin cross section stained with toluidine blue the regenerated myelinated fibers have not yet been identified within the medical device, both at a proximal and at a distal level; however, few positive neurofilament fibers were appreciated with immunohistochemical analysis.

On the contrary, the nerve repaired with the tubular membrane comprising P3HB and silver nanowires showed an early regeneration, in fact after 2 weeks from the implantation it was already possible to observe the formation of regenerated fibers in the proximal stump. Cellular colonization was observed in the distal stump.

The nerves repaired by autotransplantation showed many axons that were still degenerating. A few regenerating fibers with a thin myelin sheath were identified.

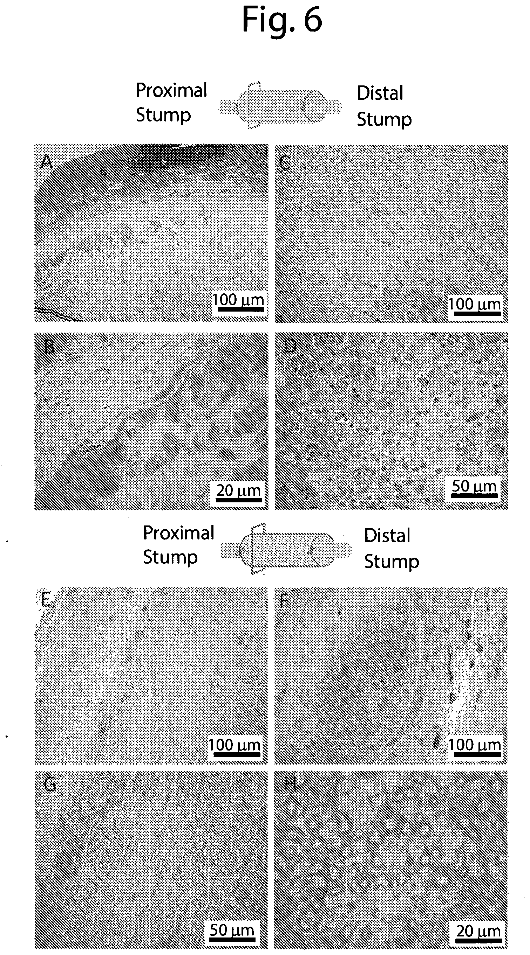

Figure 6 shows, by comparison, images of a nerve repaired with the tubular membrane comprising P3HB (without Ag) and of a nerve repaired with the tubular membrane comprising P3HB enriched with silver nanowires, the images were obtained by optical microscopy. In particular, the images represent semi-thin transversal sections stained with toluidine blue of the proximal portion of a median nerve repaired with the medical device comprising P3HB (without Ag) (A-D) and of the proximal portion of a median nerve repaired with the medical device comprising P3HB enriched with silver nanowires (E-H), 2 weeks after implantation.

### (j) Four weeks after the surgery.

The trichrome staining of the longitudinal sections showed the presence of a thin layer of connective tissue surrounding the outer side of the medical device comprising a tubular membrane comprising P3HB even after 4 weeks of nerve repair. Furthermore, it was possible to identify a rich cellular colonization, especially in transversal sections stained with toluidine blue. Unlike the results 2 weeks after surgery, in which the central portion of the medical device was still empty, after 4 weeks the entire tubular membrane comprising P3HB was colonized. Many blood vessels, even of large diameter, were easily identified with a toluidine blue staining.

Interestingly, after 4 weeks, many regenerated fibers are present in the proximal portion of the cell membrane. The regenerated fibers were easily identified both by immunofluorescence analysis, showing many positive longitudinal neurofilament fibers surrounded by Schwann positive S-100 cells, and by high resolution optical microscopy, showing many transverse regenerating fibers with their myelin sheath.

Many transverse regeneration fibers can also be identified, 4 weeks after implants, in the proximal nerve stump repaired the tubular membrane comprising P3HB enriched with silver nanowires.

In the distal stump of these repaired nerves, it is possible to observe the formation of endoneural tubes, which will guide the regenerated fibers towards the target organs.

The nerves regenerated by autotransplantation showed many myelinated fibers both proximally and distally. Few degeneration fibers have been identified.

### (k) Conclusions of the pre-clinical study.

All tubular membranes comprising P3HB (both with and without Ag nanowires) were found at the implant site, covered with a thin layer of connective tissue. The careful examination of the peri-implantation site shows the absence of signs of inflammation or scar tissue formation, demonstrating the biocompatibility of these membranes. From the macro and microscopic analysis, differences have emerged regarding the regeneration pattern. In particular, it was observed that in the tubular membranes comprising P3HB enriched with Ag nanowires, the nerve regeneration occurs near the duct wall, while in the tubular membranes without Ag nanowires the regenerating axons are located in the center of the medical device itself. Moreover, through the optical microscopy analysis of semi-thin sections, it was possible to observe that the tubular membranes comprising P3HB and Ag nanowires showed regenerating nerve fibers already two weeks after implantation, thus demonstrating an earlier regeneration compared to the membranes without Ag nanowires. From these analyzes it is therefore possible to conclude that the Ag nanowires present in the membrane are capable of attracting the regenerating nerve fibers, providing a support pathway that facilitates the correct orientation thereof: from proximal to distal. The correct orientation of the axons during the regeneration phase leads to a faster nerve regeneration which, as a result, involves an early re-innervation of the target muscle tissue and consequently an early motor recovery.

## Claims

1. Medical device comprising a tubular membrane having openings adapted to receive the ends of an injured nerve and a lumen to allow the regeneration of said nerve, wherein said tubular membrane comprises fibers of polyhydroxyalkanoate (PHA) containing 3-hydroxybutyrate monomer units, said fibers having an average diameter comprised from 500 nm to 2000 nm;
wherein the tubular membrane further comprises silver (Ag) nanowires coated by a binding agent;
wherein the binding agent is selected from:
a thiol R-SH, wherein R is a saturated or unsaturated, aliphatic and/or aromatic hydrocarbon group, having from 8 to 38 carbon atoms, optionally substituted with functional groups selected from: ester, amide, carboxyl, hydroxyl, amine groups; and
a protein biopolymer containing -SH groups.

2. Device according to claim 1, wherein the PHA fibers are oriented in a direction substantially parallel to the longitudinal axis of the tubular membrane.

3. Device according to claim 1 or 2, wherein the PHA fibers are produced by electrospinning.

4. Device according to any one of the previous claims, wherein the binding agent is selected from products of general formula (I):
HS-Ph-CO-NH-R₁-CO-OR₂ (I)
wherein:
Ph is a phenyl;
R₁ is a linear or branched C₄-C₂₀ alkylene;
R₂ is a linear or branched C₁-C₄ alkyl.

5. Device according to claim 4, wherein R₁ is a linear C₈-C₁₆ alkylene.

6. Device according to claim 4 or 5, wherein R₂ is selected from: methyl, ethyl, n-propyl, i-propyl, b-butyl, i-butyl, t-butyl.

7. Device according to any one of claims 1 to 3, wherein the binding agent is an animal gelatin.

8. Device according to any one of the previous claims, wherein the tubular membrane comprises from 0.2% to 5% by weight, preferably from 0.5% to 2.5% by weight, with respect to the overall weight of the membrane, of Ag nanowires.

9. Device according to any one of the previous claims, wherein the Ag nanowires are oriented in a direction substantially parallel to the longitudinal axis of the tubular membrane.

10. Device according to any one of the previous claims, wherein the Ag nanowires coated with the binding agent comprise from 60% to 90% by weight, preferably from 65% to 80% by weight, of Ag and from 10% to 40% by weight, preferably from 20% to 35% by weight, of the binding agent.

11. Device according to any one of the previous claims, wherein the PHA containing 3-hydroxybutyrate monomer units is a poly-3-hydroxybutyrate homopolymer (P3HB) or a copolymer containing at least 20% moles of 3-hydroxybutyrate monomer units, the remainder being hydroxyalkanoate monomer units other than 3-hydroxybutyrate.

12. Method for producing a medical device according to any one of the previous claims, which comprises:
- preparing a spinning solution by solubilization of polyhydroxyalkanoate (PHA) containing 3-hydroxybutyrate monomer units in an organic solvent;
- dispersing Ag nanowires into the spinning solution, so as to achieve a co-deposition of PHA fibers and Ag nanowires on the rotating support; wherein the Ag nanowires are previously coated by a binding agent selected from:
a thiol R-SH, wherein R is a saturated or unsaturated, aliphatic and/or aromatic hydrocarbon group, having from 8 to 38 carbon atoms, optionally substituted with functional groups selected from: ester, amide, carboxyl, hydroxyl, amine groups; and
a protein biopolymer containing -SH groups;
- subjecting the spinning solution to an electrospinning process on a rotating support so as to obtain a tubular membrane.

13. Method according to claim 12, wherein the solvent is selected from 1,1,1,3,3,3-hexafluoro-2-propanol (HFIP), chloroform, N,N-dimethylformamide (DMF), or mixtures thereof.

14. Method according to claim 12 or 13, wherein in the spinning solution, the PHA containing 3-hydroxybutyrate monomer units has a concentration comprised from 1% to 20% w/v, preferably comprised from 2 to 10% w/v.

15. Method according to any one of claims 12 to 14, wherein the Ag nanowires are present in the spinning solution in a concentration comprised from 0.05% to 2.0% w/v, preferably comprised from 0.1% to 1.0% w/v.

## Patentansprüche

1. Medizinische Vorrichtung, umfassend eine röhrenförmige Membran mit Öffnungen, die geeignet sind, die Enden eines verletzten Nervs aufzunehmen, und einem Lumen, um die Regeneration des Nervs zu ermöglichen, wobei die röhrenförmige Membran Fasern aus Polyhydroxyalkanoat (PHA) umfasst, die 3-Hydroxybutyrat-Monomereinheiten enthalten, wobei die Fasern einen durchschnittlichen Durchmesser von 500 nm bis 2000 nm aufweisen; wobei die röhrenförmige Membran ferner Silber(Ag)-Nanodrähte umfasst, die mit einem Bindemittel beschichtet sind; wobei das Bindemittel ausgewählt ist aus:
ein Thiol R-SH, worin R eine gesättigte oder ungesättigte, aliphatische und/oder aromatische Kohlenwasserstoffgruppe mit 8 bis 38 Kohlenstoffatomen ist, die gegebenenfalls mit funktionellen Gruppen substituiert ist, die ausgewählt sind aus: Ester-, Amid-, Carboxyl-, Hydroxyl- und Amingruppen; und
ein Protein-Biopolymer mit -SH-Gruppen.

2. Vorrichtung nach Anspruch 1, wobei die PHA-Fasern in einer Richtung ausgerichtet sind, die im Wesentlichen parallel zur Längsachse der röhrenförmigen Membran verläuft.

3. Vorrichtung nach Anspruch 1 oder 2, wobei die PHA-Fasern durch Elektrospinnen hergestellt werden.

4. Vorrichtung nach einem der vorstehenden Ansprüche, **dadurch gekennzeichnet, dass** das Bindemittel ausgewählt ist aus Produkten der allgemeinen Formel (I):
HS-Ph-CO-NH-R₁-CO-OR₂ (I)
wobei:
Ph ein Phenyl ist;
R₁ ein lineares oder verzweigtes C₄-C₂₀ -Alkylen ist;
R₂ ein lineares oder verzweigtes C₁-C₄ -Alkyl ist.

5. Vorrichtung nach Anspruch 4, wobei R₁ ein lineares C₈-C₁₆ -Alkylen ist.

6. Vorrichtung nach Anspruch 4 oder 5, wobei R2 ausgewählt ist aus: Methyl, Ethyl, n-Propyl, i-Propyl, b-Butyl, i-Butyl, t-Butyl.

7. Vorrichtung nach einem der Ansprüche 1 bis 3, wobei das Bindemittel eine tierische Gelatine ist.

8. Vorrichtung nach einem der vorstehenden Ansprüche, wobei die röhrenförmige Membran 0,2 bis 5 Gew.-%, vorzugsweise 0,5 bis 2,5 Gew.-%, bezogen auf das Gesamtgewicht der Membran, an Ag-Nanodrähten umfasst.

9. Vorrichtung nach einem der vorstehenden Ansprüche, wobei die Ag-Nanodrähte in einer Richtung ausgerichtet sind, die im Wesentlichen parallel zur Längsachse der röhrenförmigen Membran verläuft.

10. Vorrichtung nach einem der vorstehenden Ansprüche, wobei die mit dem Bindemittel beschichteten Ag-Nanodrähte 60 bis 90 Gew.-%, vorzugsweise 65 bis 80 Gew.-%, Ag und 10 bis 40 Gew.-%, vorzugsweise 20 bis 35 Gew.-%, des Bindemittels umfassen.

11. Vorrichtung nach einem der vorstehenden Ansprüche, wobei das PHA, das 3-Hydroxybutyrat-Monomereinheiten enthält, ein Poly-3-hydroxybutyrat-Homopolymer (P3HB) oder ein Copolymer ist, das mindestens 20 % Mole von 3-Hydroxybutyrat-Monomereinheiten enthält, wobei der Rest andere Hydroxyalkanoat-Monomereinheiten als 3-Hydroxybutyrat sind.

12. Verfahren zur Herstellung einer medizinischen Vorrichtung nach einem der vorstehenden Ansprüche, welches Folgendes umfasst:
- Herstellen einer Spinnlösung durch Solubilisierung von Polyhydroxyalkanoat (PHA), das 3-Hydroxybutyrat-Monomereinheiten in einem organischen Lösungsmittel enthält;
- Dispergieren von Ag-Nanodrähten in die Spinnlösung, um eine Co-Abscheidung von PHA-Fasern und Ag-Nanodrähten auf dem rotierenden Träger zu erreichen; wobei die Ag-Nanodrähte zuvor mit einem Bindemittel beschichtet werden, das ausgewählt ist aus:
einem Thiol R-SH, worin R eine gesättigte oder ungesättigte, aliphatische und/oder aromatische Kohlenwasserstoffgruppe mit 8 bis 38 Kohlenstoffatomen ist, die gegebenenfalls mit funktionellen Gruppen substituiert ist, die ausgewählt sind aus: Ester-, Amid-, Carboxyl-, Hydroxyl- und Amingruppen; und einem Proteinbiopolymer, das -SH-Gruppen enthält;
- Unterziehen der Spinnlösung einem Elektrospinnverfahren auf einem rotierenden Träger, um eine röhrenförmige Membran zu erhalten.

13. Verfahren nach Anspruch 12, wobei das Lösungsmittel ausgewählt ist aus 1,1,1,3,3,3-Hexafluor-2- propanol (HFIP), Chloroform, N,N-Dimethylformamid (DMF) oder Mischungen davon.

14. Verfahren nach Anspruch 12 oder 13, wobei in der Spinnlösung das PHA, das 3-Hydroxybutyrat-Monomereinheiten enthält, eine Konzentration von 1 % bis 20 % Gewicht/Volumen, vorzugsweise von 2 bis 10 % Gewicht/Volumen aufweist.

15. Verfahren nach einem der Ansprüche 12 bis 14, wobei die Ag-Nanodrähte in der Spinnlösung in einer Konzentration von 0,05 % bis 2,0 % Gewicht/Volumen, vorzugsweise von 0,1 % bis 1,0 % Gewicht/Volumen, vorhanden sind.

## Revendications

1. Dispositif médical comprenant une membrane tubulaire ayant des ouvertures conçues pour recevoir les extrémités d'un nerf lésé et une lumière pour permettre la régénération dudit nerf, dans lequel ladite membrane tubulaire comprend des fibres de polyhydroxyalcanoate (PHA) contenant des unités monomère 3-hydroxybutyrate, lesdites fibres ayant un diamètre moyen compris de 500 nm à 2000 nm; dans lequel la membrane tubulaire comprend en outre des nanofils d'argent (Ag) recouverts par un agent de liaison; dans lequel l'agent de liaison est sélectionné parmi:
un thiol R-SH, dans lequel R est un groupe hydrocarbure aliphatique et/ou aromatique, saturé ou insaturé, ayant 8 à 38 atomes de carbone, facultativement substitué par des groupes fonctionnels sélectionnés parmi : les groupes ester, amide, carboxyle, hydroxyle, amine; et
un biopolymère protéique contenant des groupes -SH.

2. Dispositif selon la revendication 1, dans lequel les fibres de PHA sont orientées dans une direction sensiblement parallèle à l'axe longitudinal de la membrane tubulaire.

3. Dispositif selon la revendication 1 ou 2, dans lequel les fibres de PHA sont produites par électrofilage.

4. Dispositif selon l'une quelconque des revendications précédentes, dans lequel l'agent de liaison est sélectionné parmi les produits de formule générale (I):
HS-Ph-CO-NH-R₁-CO-OR₂ (I)
dans laquelle:
Ph est un phényle;
R₁ est un alkylène en C₄ à C₂₀ linéaire ou ramifié;
R₂ est un alkyle en C₁ à C₄ linéaire ou ramifié.

5. Dispositif selon la revendication 4, dans lequel R₁ est un alkylène en C₈ à C₁₆ linéaire.

6. Dispositif selon la revendication 4 ou 5, dans lequel R₂ est sélectionné parmi : méthyle, éthyle, n-propyle, i-propyle, b-butyle, i-butyle, t-butyle.

7. Dispositif selon l'une quelconque des revendications 1 à 3, dans lequel l'agent de liaison est une gélatine animale.

8. Dispositif selon l'une quelconque des revendications précédentes, dans lequel la membrane tubulaire comprend de 0,2 % à 5 % en poids, de préférence de 0,5 % à 2,5 % en poids, par rapport au poids global de la membrane, de nanofils d'Ag.

9. Dispositif selon l'une quelconque des revendications précédentes, dans lequel les nanofils d'Ag sont orientés dans une direction sensiblement parallèle à l'axe longitudinal de la membrane tubulaire.

10. Dispositif selon l'une quelconque des revendications précédentes, dans lequel les nanofils d'Ag revêtus avec l'agent de liaison comprennent de 60 % à 90 % en poids, de préférence de 65 % à 80 % en poids, d'Ag et de 10 % à 40 % en poids, de préférence de 20 % à 35 % en poids, de l'agent de liaison.

11. Dispositif selon l'une quelconque des revendications précédentes, dans lequel le PHA contenant des unités monomère 3-hydroxybutyrate est un homopolymère de poly-3-hydroxybutyrate (P3HB) ou un copolymère contenant au moins 20 % en moles d'unités monomère 3-hydroxybutyrate, le reste étant des unités monomère hydroxyalcanoate autres que 3-hydroxybutyrate.

12. Procédé pour la production d'un dispositif médical selon l'une quelconque des revendications précédentes, qui comprend:
- la préparation d'une solution de filage par la solubilisation de polyhydroxyalcanoate (PHA) contenant des unités monomère 3-hydroxybutyrate dans un solvant organique;
- la dispersion de nanofils d'Ag dans la solution de filage, de façon à parvenir à un dépôt conjoint de fibres de PHA et de nanofils d'Ag sur le support rotatif ; dans lequel les nanofils d'Ag sont préalablement revêtus par un agent de liaison sélectionné parmi:
un thiol R-SH, dans lequel R est un groupe hydrocarbure aliphatique et/ou aromatique, saturé ou insaturé, ayant 8 à 38 atomes de carbone, facultativement substitué par des groupes fonctionnels sélectionnés parmi: les groupes ester, amide, carboxyle, hydroxyle, amine; et un biopolymère protéique contenant des groupes -SH;
- la soumission de la solution de filage à un processus d'électrofilage sur un support rotatif de façon à obtenir une membrane tubulaire.

13. Procédé selon la revendication 12, dans lequel le solvant est sélectionné parmi le 1,1,1,3,3,3-hexafluoro-2-propanol (HFIP), le chloroforme, le N,N-diméthylformamide (DMF), ou les mélanges de ceux-ci.

14. Procédé selon la revendication 12 ou 13, dans lequel, dans la solution de filage, le PHA contenant des unités monomère 3-hydroxybutyrate possède une concentration comprise de 1 % à 20 % p/v, de préférence comprise de 2 à 10 % p/v.

15. Procédé selon l'une quelconque des revendications 12 à 14, dans lequel les nanofils d'Ag sont présents dans la solution de filage à une concentration comprise de 0,05 % à 2,0 % p/v, de préférence comprise de 0,1 % à 1,0 % p/v.
